(19) Europäisches Patentamt
European Patent Office
Office européen des brevets

(11) EP 0 773 016 B1

(12) **FASCICULE DE BREVET EUROPEEN**

(45) Date de publication et mention de la délivrance du brevet: **01.04.1998 Bulletin 1998/14**

(51) Int. Cl.$^6$: **A61K 7/48**, A61K 7/06

(21) Numéro de dépôt: **96401860.0**

(22) Date de dépôt: **29.08.1996**

(54) **Composition topique comprenant l'association d'un polymère à squelette non-siliconé à greffons siliconés et d'un polymère à squelette polysiloxanique à greffons non-siliconés**

Topische Zusammensetzung enthaltend die Kombination eines siloxangepfropften silikatfreien Polymers und eines silikatfrei gepfropften Polysiloxane-Polymers

Topical composition containing a combination of a silicon grafted polymer with a non-silicon backbone and a non-silicon grafted polysiloxan polymer

(84) Etats contractants désignés:
**DE FR GB**

(30) Priorité: **29.09.1995 FR 9511487**

(43) Date de publication de la demande:
**14.05.1997 Bulletin 1997/20**

(73) Titulaire: **L'OREAL**
**75008 Paris (FR)**

(72) Inventeurs:
- **Dubief, Claude**
  **78150 Le Chesnay (FR)**
- **Dupuis, Christine**
  **75018 Paris (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 388 582**
**EP-A- 0 412 710**
**WO-A-93/23446**
**WO-A-95/00108**
**WO-A-95/03776**
**WO-A-95/05800**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 773 016 B1

## Description

La présente invention a trait à une composition cosmétique ou dermatologique pour le traitement des matières kératiniques, en particulier des cheveux humains, comprenant au moins un polymère à squelette organique non-siliconé greffé par des monomères contenant un polysiloxane et au moins un polymère à squelette polysiloxanique greffé par des monomères organiques non-siliconés ainsi qu'à ses utilisations.

On connaît dans l'état de la technique des polymères à squelette organique non-siliconé greffé par des monomères contenant un polysiloxane tels que ceux décrits dans les brevets US 4,693,935 et US 4,728,571 et les demandes de brevet EP-A-0388582, EP-A-0412704, EP-A-0412707, EP-A-0412710, EP-A-0640105 (WO93/23446) et WO 95/00578. Ces polymères sont proposés en capillaire pour leurs propriétés coiffantes. On connaît également dans l'état de la technique des polymères à squelette polysiloxanique greffé par des monomères organiques non-siliconés sont choisis préférentiellement parmi ceux décrits dans les demandes EP-A-0582152 et WO 93/23009. Ils sont également proposés en capillaire pour leur propriétés coiffantes.

La demanderesse a découvert de façon surprenante qu'en associant au moins un polymère à squelette organique non-siliconé greffé par des monomères contenant un polysiloxane avec au moins un polymère à squelette polysiloxanique greffé par des monomères organiques non-siliconés, on obtenait des propriétés coiffantes supérieures à celles obtenues par chaque polymère utilisé seul ; ceci se traduit par une meilleure adhésion interfibres. La demanderesse a découvert également que l'association de polymères greffés siliconés de l'invention améliorait la douceur au toucher après application par rapport à chaque polymère de l'association utilisé seul.

La composition selon l'invention est donc essentiellement caractérisée par le fait qu'elle contient dans un milieu cosmétiquement ou dermatologiquement acceptable au moins un polymère à squelette organique non-siliconé greffé par des monomères contenant un polysiloxane et au moins un polymère à squelette polysiloxanique greffé par des monomères organiques non-siliconés.

Dans ce qui suit, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en $C_1$-$C_{10}$ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxys ou acyloxyalkyles, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Dans ce qui suit, on entend désigner par ⟨⟨macromère polysiloxane⟩⟩, en conformité avec l'acceptation générale, tout monomère contenant dans sa structure une chaîne polymère du type polysiloxane.

Les polymères à squelette organique non-siliconé greffé par des monomères contenant un polysiloxane conformes à la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisée sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935 et US

4,728,571 et les demandes de brevet EP-A-0382582, EP-A-0412704, EP-A-0412707, EP-A-0412710, EP-A-0640105 (WO93/23446) et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :

a) de 0 à 98% en poids d'au moins un monomère(A) lipophile de faible polarité lipophile à insaturation éthylénique, polymérisable par voie radicalaire ;
b) de 0 à 98% en poids d'au moins un monomère (B) hydrophile polaire à insaturation éthylénique, copolymérisable avec le ou les monomères du type (A)
c) de 0,01 à 50 % en poids d'au moins un macromère polysiloxane (C) de formule générale :

$$X(Y)_nSi(R)_{3-m}\,Z_m \qquad (I)$$

où :

X désigne un groupe vinylique copolymérisable avec les monomères (A) et (B) ;
Y désigne un groupe de liaison divalent ;
R désigne un hydrogène, un alkyle ou un alcoxy en $C_1$-$C_6$, un aryle $C_6$-$C_{12}$ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,693,935, US 4,728,571 et US 4,972,037 et les demandes de brevet EP-A-0412704, EP-A-0412707, EP-A-0640105. Ils ont un poids moléculaire moyen en nombre de préférence allant de 10.000 à 2.000.000 et de préférence une température de transition vitreuse Tg ou une température de fusion cristalline Tm d'au moins - 20°C.

On peut citer comme exemples de monomères lipophiles (A), les esters d'acide acrylique ou méthacrylique d'alcools en $C_1$-$C_{18}$ ; le styrène ; les macromères polystyrène ; l'acétate de vinyle ; le propionate de vinyle ; l'alpha-méthylstyrène ; le tertiobutylstyrène ; le butadiène ; le cyclohexadiène ; l'éthylène ; le propylène ; le vinyltoluène ; les esters d'acide acrylique ou méthacrylique et de 1,1-dihydro-perfluoroalcanol ou de ses homologues ; les esters d'acide acrylique ou méthacrylique et de oméga-hydrydofluroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluroéther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perflurooctane sulfonamido)-éthylacrylate ; le 2-(N-butylperflurooctane sulfonamido)-éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maleique, l'anhydride maléique et leurs demi-esters, les (méth) acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (II) :

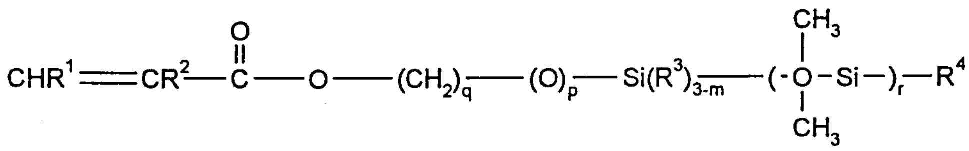

(II)

dans laquelle :

$R^1$ est hydrogène ou -COOH (de préférence hydrogène) ;
$R^2$ est hydrogène, méthyle ou $-CH_2COOH$ (de préférence méthyle) ;
$R^3$ est alkyle, alcoxy ou alkylamino en $C_1$-$C_6$, aryle en $C_6$-$C_{12}$ ou hydroxyle (de préférence méthyle) ;
$R^4$ est alkyle, alcoxy ou alkylamino en $C_1$-$C_6$, aryle en $C_6$-$C_{12}$ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1).

On utilise plus particulièrement les macromères polysiloxanes de formule :

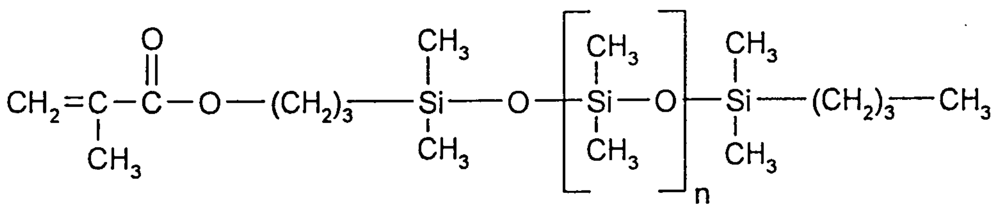

avec n étant un nombre allant de 5 à 700.

Un mode particulier de réalisation de l'invention consiste à utiliser un copolymère susceptible d'être obtenu par polymérisation radicalaire à partir du mélange de monomères constitué par :

a) 60% en poids d'acrylate de tertiobutyle ;

b) 20% en poids d'acide acrylique ;

c) 20% en poids de macromère siliconé de formule :

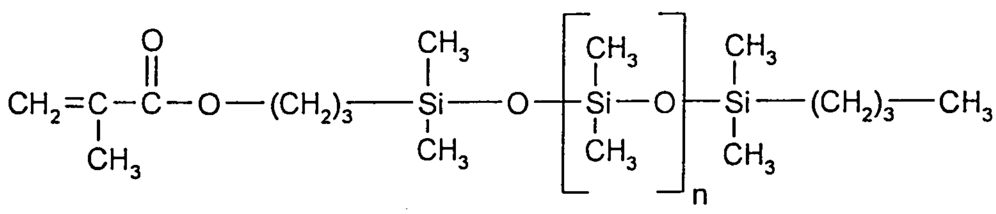

avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :

a) 80% en poids d'acrylate de tertiobutyle ;
b) 20% en poids de macromère siliconé de formule :

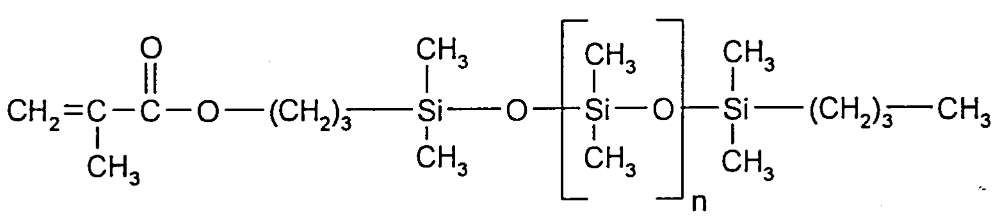

avec n étant un nombre allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkylstyrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (III):

$$T\text{-}(CH_2)_s\text{-}Si\text{-}[\text{-}(OSiR^5R^6)_t\text{-}R^7]_y \qquad (III)$$

dans laquelle T est choisi dans le groupe constitué par NH2, NHR', une fonction époxy, OH, SH ; $R^5$, $R^6$, $R^7$ et R', indépendamment, désignent un alkyle en $C_1$-$C_6$, phenyle, benzyle, ou alkylphényle en $C_6$-$C_{12}$, hydrogène ; s est un nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9.000 à 40.000.

Selon la présente invention, le ou les polymères à squelette polysiloxanique greffé par des monomères organiques non-siliconés comprennent une chaîne principale de silicone (ou polysiloxane $(\equiv Si\text{-}O\text{-})_n$) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements $\equiv$Si-H et des groupements vinyliques $CH_2$=CH-, ou encore la réaction entre des groupements thio-fonctionnels -SH avec ces mêmes groupements vinyliques.

Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth) acrylate de méthyle, le (méth)acrylate de tertio-butyle, le (méth)acrylate de tridécyle, le (méth)acrylate de stéaryle ou leurs mélanges.

Une famille de polymères à squelette polysiloxanique greffé par des monomères organiques non-siliconés convenant particulièrement bien à la mise en oeuvre de la présente invention est constituée par les polymères siliconés comportant dans leur structure le motif de formule (I) suivant :

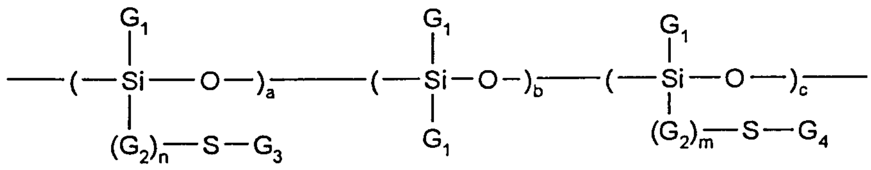

(IV)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo) polymérisation d'au moins un monomère hydrophobe à insaturation éthylènique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :

- les radicaux $G_1$ désignent un radical alkyle, de préférence le radical méthyle ;
- n est non nul, et les radicaux $G_2$ représentent un radical divalent en $C_1$-$C_3$, de préférence un radical propylène ;
- $G_3$ représente un radical polymérique résultant de l'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth)acrylate d'alkyle($C_1$-$C_{10}$), de préférence le (méth)acrylate d'isobutyle ou de méthyle.

Des exemples de polymères siliconés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly (méth)acrylique et du type poly(méth)acrylate de méthyle.

D'autres exemples de polymères siliconés répondant à la formule (IV) sont notamment des polydiméthylsiloxanes (PDMS) sur lesquels sont greffés, par l'intermédiaire d'un chaînon de raccordement de type thiopropylène, des motifs polymères du type poly (méth)acrylate d'isobutyle.

De préférence, la masse moléculaire en nombre des polymères à squelette polysiloxanique greffé par des monomères organiques non-siliconés de l'invention varie de 10 000 à 1 000 000 environ, et encore plus préférentiellement de 10 000 à 100 000 environ.

Les polymères à squelette organique non-siliconé greffé par des monomères contenant un polysiloxane sont utilisés de préférence en une quantité allant de 0,01 à 15% en poids du poids total de la composition. Encore plus préférentiellement, cette quantité varie de 0,5 à 10% en poids.

Les polymères à squelette polysiloxanique greffé par des monomères organiques non-siliconés, sont utilisés de préférence en une quantité allant de 0,01 à 20% en poids du poids total de la composition. Plus préférentiellement, cette quantité varie de 0,1 à 15% en poids et encore plus préférentiellement de 0,5 à 10% en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement non-thérapeutique des matières kératiniques telles que les cheveux consistant à appliquer sur celles-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

EXEMPLES

| **EXEMPLE 1 Spray de coiffage en flacon pompe** | |
|---|---|
| - Polymère siliconé greffé de formule (IV) de structure polyméthyl/méthylsiloxane à groupements propyl thio-3 acide polyméthacrylique et groupements propyl thio-3 polyméthacrylate de méthyle | 3 g en MA |
| - Polymère siliconé greffé de structure (1) telle que définie ci-dessous | 3 g en MA |
| - Aminométhylpropanol neutralisation à 100% des deux polymères siliconés greffés qsp | |
| - Ethanol qsp | 100 g |

Structure (1) :

Copolymère obtenu par polymérisation radicalaire à partir du mélange de monomères constitué par :

a) 60% en poids d'acrylate de tertiobutyle ;
b) 20% en poids d'acide acrylique ;
c) 20% en poids de macromère siliconé de formule :

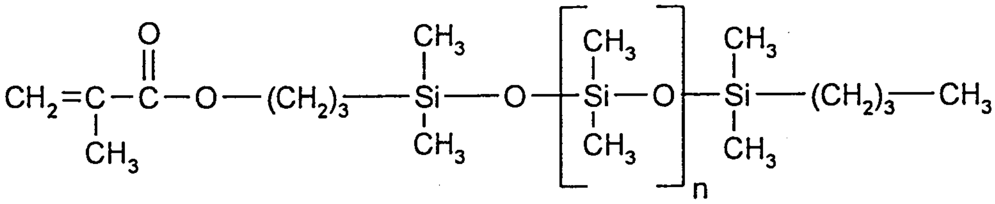

avec n étant un nombre choisi de telle sorte que le poids moléculaire moyen en nombre du macromère soit compris entre environ 9.000 et 12.000 ; les pourcentages en poids étant calculés par rapport au poids total des monomères.

| **<u>EXEMPLE 2</u> Spray aérosol** | |
|---|---|
| - Polymère siliconé greffé de formule (IV) de structure polyméthyl/méthylsiloxane à groupements propyl propyl thio-3 acide polyméthacrylique et propyl thio-3 polyméthacrylate de méthyle | 3,75 g en MA |
| - Polymère siliconé greffé de structure (1) telle que définie ci-dessus | 3,75 g en MA |
| - Aminométhylpropanol neutralisation à 100% des deux polymères siliconés greffés qsp | |
| -Ethanol qsp | 100 g |

| Schéma de pressurisation : | |
|---|---|
| - Composition ci-dessus | 80 g |
| - Isobutane | 15 g |
| - 1,1-difluoroéthane | 5 g |

| **<u>EXEMPLE 3</u> Gel de coiffage** | |
|---|---|
| - Polymère siliconé greffé de formule (IV) de structure polyméthyl/méthylsiloxane à groupements propyl thio-3 acidepolyméthacrylique et groupements propyl thio-3 polyméthacrylate de méthyle | 1 g en MA |
| - Polymère siliconé greffé de structure (1) telle que définie ci-dessus | 1 g en MA |
| - Poly(acide acrylique) réticulé vendu sous le nom SYNTHALEN K par la société 3V | 0,5 g en MA |
| - Aminométhylpropanol qsp | pH 7,5 |
| - Eau déminéralisée qsp | 100 g |

| **EXEMPLE 4** **Mousse de coiffage** | |
|---|---|
| - Polymère siliconé greffé de formule (IV) de structure polyméthyl/méthylsiloxane à groupements propyl thio-3 acide polyméthacrylique et groupements propyl thio-3 polyméthacrylate de méthyle | 1 g en MA |
| - Polymère siliconé greffé de structure (1) : telle que définie ci-dessus | 1 g en MA |
| - Terpolymère acide acrylique/vinylpyrrolidone/méthacrylate de lauryle (68/23/9%) vendu sous le nom ACRYLIDONE LM par la société ISP | 0,5 g en MA |
| - Aminométhylpropanol qsp | pH 7,5 |
| - Eau déminéralisée qsp | 100 g |

| Schéma de pressurisation : | |
|---|---|
| - Composition ci-dessus | 90 g |
| - Mélange isobutane/propane/butane (23/55/22) vendu sous le nom AEROGAZ 3,2 N par la Société ELF AQUITAINE | 10 g |

## EXEMPLES COMPARATIFS

(I) EXEMPLES COMPARATIFS SUR LE POUVOIR FIXANT

On a étudié et comparé le pouvoir fixant des trois formulations A, B et C suivantes sur les cheveux :

| COMPOSITION A (art antérieur) : | |
|---|---|
| - Polymère $P_1$ siliconé greffé de formule (IV) de structure polyméthyl/méthylsiloxane à groupements propyl thio-3 acide polyméthacrylique et groupements propyl thio-3 polyméthacrylate de méthyle | 5 g en MA |
| - Monométhyléthertripopylèneglycol (plastifiant) | 0,5 g |
| - Aminométhylpropanol neutralisation à 100% du polymère siliconé greffé qsp | |
| - Ethanol à 98,5% qsp | 100 g |

| COMPOSITION B (art antérieur) : | |
|---|---|
| - Polymère $P_2$ siliconé greffé de structure (1) telle que définie ci-dessus | 1 g en MA |
| - Monométhyléthertripopylèneglycol (plastifiant) | 0,1 g |
| - Aminométhylpropanol neutralisation à 100% du polymère siliconé greffé qsp | |
| - Ethanol à 98,5% qsp | 100 g |

| COMPOSITION C (invention) : | |
|---|---|
| - Polymère $P_1$ siliconé greffé de formule (IV) de structure polyméthyl/méthylsiloxane à groupements propyl thio-3 acide polyméthacrylique et groupements propyl thio-3 polyméthacrylate de méthyle | 2,5 g en MA |
| - Polymère $P_2$ siliconé greffé de structure (1) telle que définie ci-dessus | 2,5 g en MA |
| - Monométhyléthertripopylèneglycol (plastifiant) | 0,5 g |
| - Aminométhylpropanol neutralisation à 100% des polymères siliconés greffés qsp | |
| - Ethanol à 98,5% qsp | 100 g |

MODE OPERATOIRE

On effectue un test de mesure de la force des liaisons formées entre les cheveux par une laque capillaire selon les principes de la méthode décrite dans l'article de R. RANDALL WICKETT, JOHN A. SRAMEK et CYNTHIA M. TROBAUGH dans la revue Journ. Soc. Cosmet. Chem. 43, 169-178 (Mai/Juin 1992).

Pour chaque formulation testée, on prend un cheveu unitaire. On effectue sur le cheveu une simple boucle ayant un diamètre de 2 cm approximativement, au moyen d'un support cylindrique. On trempe le cheveu ainsi bouclé dans la formulation et on le laisse sécher sous atmosphère conditionnée (20°C et 50% d'humidité). On coupe la boucle fixée par la formulation A, B, ou C. On obtient ainsi 2 demi-cheveux liés entre eux par un point de fixation.

On fixe les extrémités, situées de part et d'autre du point de fixation, à chacune des 2 mâchoires d'un appareil du type INSTRON ® mesurant la force de tension en Newtons exercée sur les demi-cheveux.

On mesure la force moyenne (sur dix essais) $F_A$, $F_B$ et $F_C$ (spécifique à la composition A, B ou C) nécessaire pour rompre le point de fixation reliant les deux demi-cheveux et formé par la formulation A, B ou C.

On détermine l'amélioration sur le pouvoir fixant apportée par l'association des polymères greffés siliconés $P_1 + P_2$ par rapport à chacun de ces polymères utilisés seul, en calculant la variation relative de la force de rupture exprimée en pourcentage mesurée selon la formule suivante :

$$(F_C - F_A / F_A) \times 100 \text{ ou } (F_C - F_B / F_B) \times 100$$

Les résultats sont indiqués dans le tableau ci-dessous :

| FORMULATION TESTEE | FORCE MOYENNE DE RUPTURE EXPRIMEE EN NEWTONS | AMELIORATION DE LA TENUE DE LA FORMULATION C PAR RAPPORT A LA FORMULATION A OU B (EN %) |
|---|---|---|
| C ($P_1$ +$P_2$) | 0,24 | - |
| A ($P_1$) | 0,09 | +166 % |
| B ($P_2$) | 0,19 | +26% |

On constate que la formulation C contenant l'association de polymères $P_1 + P_2$ conduit à pouvoir fixant des cheveux plus importante que celui obtenu avec la composition A et la composition B contenant respectivement le polymère $P_1$ seul et le polymère $P_2$ seul.

(II) ESSAIS COMPARATIFS SUR LA DOUCEUR AU TOUCHER

On a effectué également un test d'estimation sensorielle sur un panel de 5 personnes. On étudie comme critère cosmétique la douceur au toucher après application sur les cheveux de chacune des trois compositions A, B et C telles que décrites ci-dessus.

Les 5 personnes interrogées ont estimé que la composition C contenant l'association de polymères $P_1 + P_2$ apportait aux cheveux une douceur au toucher plus importante que celle obtenue avec la composition A et la composition B contenant respectivement le polymère $P_1$ seul et le polymère $P_2$ seul.

## Revendications

1. Composition cosmétique ou dermatologique destinée au traitement des matières kératiniques, caractérisée par le fait qu'elle contient dans un milieu cosmétiquement ou dermatologiquement acceptable au moins :

   (a) un polymère à squelette organique non-siliconé greffé par un macromère polysiloxane susceptible d'être obtenu par polymérisation radicalaire d'au moins un monomère à insaturation éthylénique et d'au moins un macromère polysiloxane ayant un groupe vinylique terminal ou bien par réaction d'une polyoléfine comprenant des groupes fonctionnalisés et d'un macromère polysiloxane ayant une fonction terminale réactive avec lesdits groupes fonctionnalisés.; et
   (b) au moins un polymère à squelette polysiloxanique greffé par un groupe organique non-siliconé susceptible d'être obtenu par copolymérisation radicalaire entre d'une part (i) au moins un monomère organique anionique non-siliconé présentant une insaturation éthylènique et/ou un monomère organique hydrophobe non-siliconé présentant une insaturation éthylénique et (ii) d'autre part un polysiloxane présentant dans sa chaîne au moins un ou plusieurs groupements fonctionnels capables de venir réagir sur lesdites insaturations éthyléniques desdits monomères non-siliconés.

2. Composition selon la revendication 1, caractérisée par le fait que le polymère siliconé greffé à squelette organique non-siliconé est un copolymère comprenant :

   a) de 0 à 98% en poids d'au moins un monomère(A) lipophile de faible polarité à insaturation éthylénique de faible polarité, polymérisable par voie radicalaire ;
   b) de 0 à 98% en poids d'au moins un monomère (B) hydrophile polaire à insaturation éthylénique, copolymérisable avec le ou les monomères du type (A) ;
   c) de 0,01 à 50 % en poids d'au moins un macromère polysiloxane (C) de formule générale :

   $$X(Y)_nSi(R)_{3-m}\text{-}Z_m \qquad (I)$$

   où :

   X désigne un groupe vinylique copolymérisable avec les monomères (A) et (B) ;
   Y désigne un groupe de liaison divalent ;
   R désigne un hydrogène, un alkyle ou un alcoxy en $C_1$-$C_6$, un aryle $C_6$-$C_{12}$ ;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

3. Composition selon la revendication 2, caractérisée par le fait que les monomères lipophiles (A) sont choisis dans le groupe constitué par les esters d'acide acrylique ou méthacrylique d'alcools en $C_1$-$C_{18}$ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydo-fluroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluoroéther d'alcool ; ou leurs mélanges.

4. Composition selon la revendication 3, caractérisée par le fait que les monomères lipophiles (A) sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-butylperfluorooctane sulfonamido)-éthylacrylate, le 2-(N-méthylperfluorooctane sulfonamido)-éthylacrylate et leurs mélanges.

5. Composition selon l'une quelconque des revendications 2 à 4, caractérisée par le fait que les monomères polaires (B) sont choisis dans le groupe constitué par : l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maleique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique,

l'alcool vinylique, le vinyl caprolactame ou leurs mélanges.

6. Composition selon la revendication 5, caractérisée par le fait que les monomères polaires (B) sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

7. Composition selon l'une quelconque des revendications 2 à 6, caractérisée par le fait que le macromère polysiloxane (C) correspond à la formule générale suivante (II) :

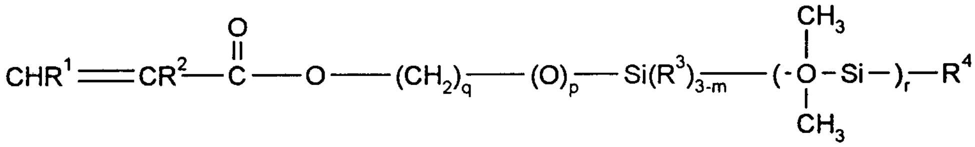

(II)

dans laquelle :

$R^1$ est hydrogène ou -COOH ;
$R^2$ est hydrogène, méthyle ou $-CH_2COOH$ ;
$R^3$ est alkyle, alcoxy ou alkylamino en $C_1$-$C_6$, aryle en $C_6$-$C_{12}$ ou hydroxyle ;
$R^4$ est alkyle, alcoxy ou alkylamino en $C_1$-$C_6$, aryle en $C_6$-$C_{12}$ ou hydroxyle ;
q est un entier de 2 à 6 ;
p est 0 ou 1 ;
r est un nombre entier de 5 à 700 ;
m est un entier allant de 1 à 3.

8. Composition selon l'une quelconque des revendications 2 à 7, caractérisée par le fait que le macromère polysiloxane (C) correspond à la formule générale suivante :

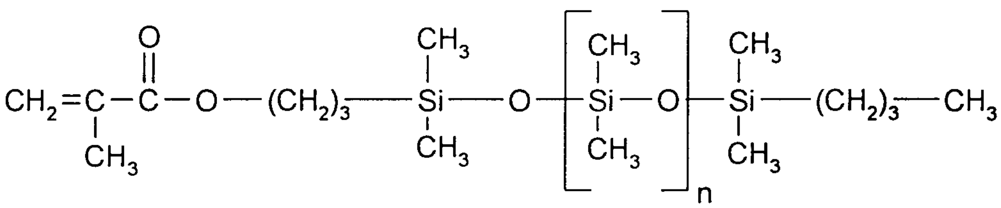

avec n étant un nombre allant de 5 à 700.

9. Composition selon l'une quelconque des revendications 1 à 8, caractérisée par le fait que le polymère siliconé greffé à squelette organique non-siliconé est un copolymère susceptible d'être obtenu par polymérisation radicalaire à partir du mélange de monomères constitué par :

a) 60% en poids d'acrylate de tertiobutyle ;
b) 20% en poids d'acide acrylique ;
c) 20% en poids de macromère siliconé de formule :

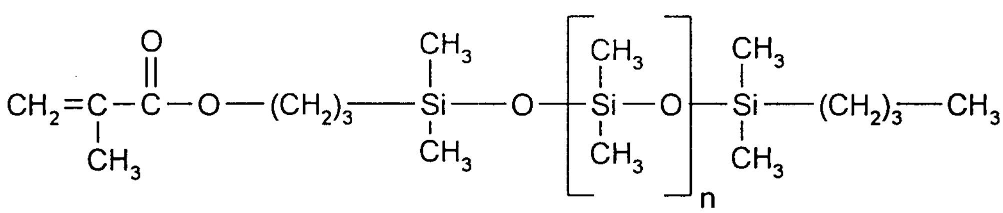

avec n étant un nombre allant de 5 à 700 ; les pourcentages en poids étant calculés par rapport au poids total

des monomères.

**10.** Composition selon l'une quelconque des revendications 1 à 8, caractérisée par le fait que le polymère siliconé greffé à squelette organique non-siliconé est un copolymère susceptible d'être obtenu par polymérisation radicalaire à partir du mélange de monomères constitué par :

a) 80% en poids d'acrylate de tertiobutyle ;
b) 20% en poids de macromère siliconé de formule :

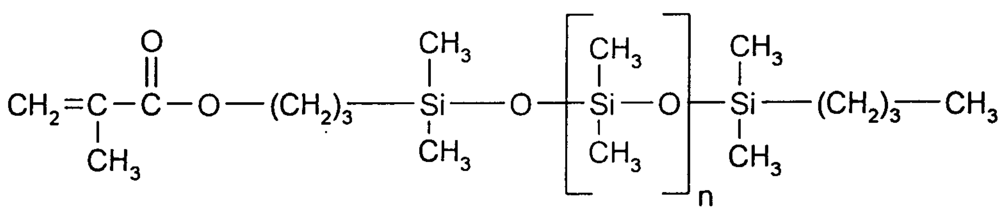

avec n étant un nombre allant de 5 à 700 ; les pourcentages en poids étant calculés par rapport au poids total des monomères.

**11.** Composition selon l'une quelconque des revendications 1 à 10, caractérisée par le fait que le polymère à squelette organique non-siliconé greffé par des monomères contenant un polysiloxane a un poids moléculaire moyen en nombre allant de 10.000 à 2.000.000 et une température de transition vitreuse Tg ou une température de fusion cristalline Tm d'au moins -20°C.

**12.** Composition selon la revendication 1, caractérisée par le fait que le polymère siliconé greffé à squelette organique non-siliconé est un polymère susceptible d'être obtenu par extrusion réactive d'un macromère polysiloxane ayant une fonction réactive terminale sur un polymère du type polyoléfine comportant des groupes réactifs susceptibles de réagir avec la fonction réactive terminale du macromère polysiloxane pour former une liaison covalente permettant le greffage de la silicone sur la chaîne principale de la polyoléfine.

**13.** Composition selon la revendication 12, caractérisée par le fait que la polyoléfine réactive est choisie dans le groupe constitué par les polyéthylènes ou les polymères de monomères dérivés de l'éthylène comportant des fonctions réactives susceptibles de réagir avec la fonction terminale du macromère polysiloxane.

**14.** Composition selon la revendication 12 ou 13, caractérisée par le fait que la polyoléfine réactive est choisie dans le groupe constitué par les copolymères d'éthylène ou de dérivés d'éthylène et de monomères choisis parmi ceux comportant une fonction carboxylique ; ceux comportant une fonction anhydride d'acide ; ceux comportant une fonction chlorure d'acide ; ceux comportant une fonction ester ; ceux comportant une fonction isocyanate.

**15.** Composition selon l'une quelconque des revendications 12 à 14, caractérisée par le fait que le macromère polysiloxane est un polysiloxane comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires.

**16.** Composition selon l'une quelconque des revendications 12 à 15, caractérisée par le fait que le macromère polysiloxane est un polysiloxane répondant à la formule générale :

$$T\text{-}(CH_2)_s\text{-}Si\text{-}[\text{-}(OSiR^5R^6)_t\text{-}R^7\,]_y \qquad (III)$$

dans laquelle T est choisi dans le groupe constitué par NH2, NHR', une fonction époxy, OH, SH ; $R^5$, $R^6$, $R^7$ et R', indépendamment, désignent un alkyle en $C_1$-$C_6$, phényle, benzyle, ou alkylphényle en $C_6$-$C_{12}$, hydrogène ; s est un nombre de 2 à 100 ; t est un nombre de 0 à 1000 et y est un nombre de 1 à 3.

**17.** Composition selon la revendication 1, caractérisée par le fait que le polymère à squelette polysiloxanique greffé par un groupe organique non-siliconé est susceptible d'être obtenu par copolymérisation radicalaire entre d'une part (i) au moins un monomère organique anionique non-siliconé à insaturation éthylénique choisi, seul ou sous forme de mélange de monomères, parmi les acides carboxyliques insaturés, linéaires ou ramifiés et/ou un monomère hydrophobe non-siliconé à insaturation éthylénique et (ii) d'autre part un polysiloxane présentant dans sa chaîne au moins un ou plusieurs groupements fonctionnels capables de venir réagir sur lesdites insaturations éthyléniques desdits monomères non-siliconés.

**18.** Composition selon la revendication 17, caractérisée par le fait que le monomère organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

**19.** Composition selon la revendication 17 , caractérisée par le fait que le monomère organique hydrophobe à insaturation éthylénique est choisi, seul ou en mélanges de monomères, parmi les esters d'acide acrylique d'alcanol et/ou les esters d'acide méthacrylique d'alcanol, de préférence l'alcanol étant en $C_1$-$C_{18}$.

**20.** Composition selon la revendication 19, caractérisée par le fait que le monomère organique hydrophobe à insaturation éthylénique est choisi, seul ou en mélange de monomères dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth) acrylate de méthyle, le (méth)acrylate de tertio-butyle, le (méth)acrylate de tridécyle, le (méth)acrylate de stéaryle.

**21.** Composition selon l'une quelconque des revendications 1, 17 et 18, caractérisée par le fait que le polymère à squelette polysiloxanique greffé comprend sur la chaîne silicone principale, au moins un groupement organique à caractère anionique obtenu par l'(homo)polymérisation radicalaire d'au moins un monomère anionique de type acide carboxylique insaturé, partiellement ou totalement neutralisé sous la forme d'un sel.

**22.** Composition selon l'une quelconque des revendications 17 à 21, caractérisée par le fait que le polymère siliconé greffé est choisi parmi les polymères siliconés comportant dans leur structure le motif de formule (IV) suivant :

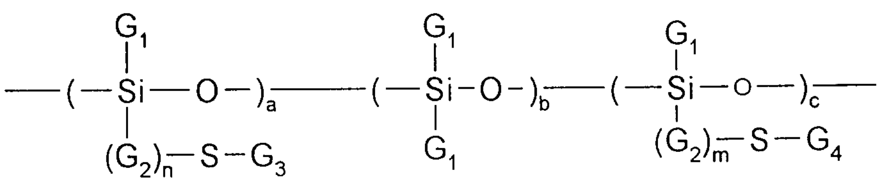

(IV)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylènique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

**23.** Composition selon la revendication 22, caractérisée par le fait que le motif de formule (IV) présente au moins l'une des caractéristiques suivantes :

- les radicaux $G_1$ désignent un radical alkyle en $C_1$-$C_{10}$ ;
- n est non nul, et les radicaux $G_2$ représentent un radical divalent en $C_1$-$C_3$ ;
- $G_3$ représente un radical polymérique résultant de l'(homo)polymérisation d'au moins un monomère du type acide carboxylique à insaturation éthylénique ;
- $G_4$ représente un radical polymérique résultant de l'(homo)polymérisation d'au moins un monomère du type (méth)acrylate d'alkyle ($C_1$-$C_{10}$).

**24.** Composition selon la revendication 22 ou 23, caractérisée par le fait que le motif de formule (IV) présente simultanément les caractéristiques suivantes :

- les radicaux $G_1$ désignent un radical méthyle ;
- n est non nul, et les radicaux $G_2$ représentent un radical propylène ;

- $G_3$ représente un radical polymérique résultant de l'(homo)polymérisation d'au moins l'acide acrylique et/ou l'acide méthacrylique ;
- $G_4$ représente un radical polymérique résultant de l'(homo)polymérisation d'au moins un monomère du type (méth)acrylate d'isobutyle ou de méthyle.

**25.** Composition selon l'une quelconque des revendications 1 à 24, caractérisée par le fait que la masse moléculaire en nombre du polymère à squelette polysiloxanique greffé par des monomères organiques non-siliconés varie de 10 000 à 1 000 000 environ, et encore plus préférentiellement de 10 000 à 100 000 environ.

**26.** Composition selon l'une quelconque des revendications 1 à 24, caractérisée par le fait que le ou les polymères à squelette organique non-siliconé greffé par des monomères contenant un polysiloxane sont utilisés en une quantité allant de 0,01 à 20% en poids par rapport au poids total de la composition et de préférence de 0,1 à 15% en poids et encore plus préférentiellement de 0,5 à 10%.

**27.** Composition selon l'une quelconque des revendications 1 à 26, caractérisée par le fait que le ou les polymères à squelette polysiloxanique greffé par des monomères organiques non-siliconés sont utilisés en une quantité allant de 0,01 à 20% en poids par rapport au poids total de la composition et de préférence de 0,1 à 15% en poids et encore plus préférentiellement de 0,5 à 10%.

**28.** Composition selon l'une quelconque des revendications 1 à 27, caractérisée par le fait qu'elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

**29.** Composition selon l'une quelconque des revendications 1 à 28, caractérisée par le fait que le milieu cosmétiquement ou dermatologiquement acceptable est constitué par de l'eau ou un mélange d'eau et d'au moins un solvant cosmétiquement acceptable.

**30.** Composition selon la revendication 29, caractérisée par le fait que les solvants cosmétiquement acceptables sont choisis dans le groupe constitué par les monoalcools, les polyalcools, les éthers de glycol, les esters d'acides gras et leurs mélanges.

**31.** Composition selon l'une quelconque des revendications 1 à 30, caractérisée par le fait que le polymère à squelette polysiloxanique greffé par des monomères organiques non-siliconés et le polymére à squelette organique non-siliconé greffé par des monomères contenant un polysiloxane peuvent être dissous dans le milieu cosmétiquement ou dermatologiquement acceptable ou bien utilisés sous forme de dispersion aqueuse de particules.

**32.** Composition selon l'une quelconque des revendications 1 à 31, caractérisée par le fait que les matières kératiniques sont des cheveux humains.

**33.** Composition selon l'une quelconque des revendications 1 à 32, caractérisée par le fait qu'elle se présente sous forme de gel, de lait, de crème, de lotion plus ou moins épaisse ou de mousse.

**34.** Composition selon l'une quelconque des revendications 1 à 33, caractérisée par le fait qu'elle est un produit de coiffage.

**35.** Composition selon l'une quelconque des revendications 1 à 33, caractérisée par le fait qu'elle est un produit capillaire choisi dans le groupe constitué par des shampooings ; des produits capillaires à rincer ou non, à appliquer avant ou après un shampooing, une coloration, une décoloration, une permanente ou un défrisage.

**36.** Composition selon l'une quelconque des revendications 1 à 35, caractérisée par le fait qu'elle est conditionnée sous forme de vaporisateur, de flacon pompe ou bien dans un récipient aérosol en vue d'obtenir un spray, une laque ou une mousse.

**37.** Procédé non-thérapeutique de traitement des matières kératiniques en particulier des cheveux, caractérisé par le fait qu'il consiste à appliquer sur lesdites matières une composition telle que définie selon l'une quelconque des revendications 1 à 36 puis à effectuer éventuellement un rinçage à l'eau.

## Claims

1. Cosmetic or dermatological composition intended for the treatment of keratinous substances, characterized in that it contains, in a cosmetically or dermatologically acceptable medium, at least:

   (a) one polymer with a non-silicone organic skeleton grafted by a polysiloxane macromer capable of being obtained by radical polymerization of at least one monomer with ethylenic unsaturation and of at least one polysiloxane macromer having an end vinyl group or else by reaction of a polyolefin comprising functionalized groups and of a polysiloxane macromer having an end functional group which reacts with the said functionalized groups; and
   (b) one polymer with a polysiloxane skeleton grafted by a non-silicone organic group capable of being obtained by radical copolymerization between, on the one hand, (i) at least one anionic non-silicone organic monomer exhibiting an ethylenic unsaturation and/or one hydrophobic non-silicone organic monomer exhibiting an ethylenic unsaturation and (ii), on the other hand, a polysiloxane exhibiting, in its chain, at least one or a number of functional groups capable of reacting with the said ethylenic unsaturations of the said non-silicone monomers.

2. Composition according to Claim 1, characterized in that the grafted silicone polymer with a non-silicone organic skeleton is a copolymer comprising:

   a) from 0 to 98 weight % of at least one lipophilic monomer (A) of low polarity with ethylenic unsaturation of low polarity which is polymerizable by the radical route;
   b) from 0 to 98 weight % of at least one polar hydrophilic monomer (B) with ethylenic unsaturation which is copolymerizable with the monomer or monomers of the type (A);
   c) from 0.01 to 50 weight % of at least one polysiloxane macromer (C) of general formula:

   $$X(Y)_nSi(R)_{3-m}\text{-}Z_m \qquad (I)$$

   where:

   X denotes a vinyl group which is copolymerizable with the monomers (A) and (B);
   Y denotes a group with divalent bonding;
   R denotes a hydrogen, a $C_1$-$C_6$ alkyl or alkoxy or a $C_6$-$C_{12}$ aryl;
   Z denotes a monovalent polysiloxane unit having a number-average molecular weight of at least 500;
   n is 0 or 1 and m is an integer ranging from 1 to 3, the percentages being calculated with respect to the total weight of the monomers (A), (B) and (C).

3. Composition according to Claim 2, characterized in that the lipophilic monomers (A) are chosen from the group composed of esters of acrylic or methacrylic acid with $C_1$-$C_{18}$ alcohols; styrene; polystyrene macromers; vinyl acetate; vinyl propionate; $\alpha$-methylstyrene; tert-butylstyrene; butadiene; cyclohexadiene; ethylene; propylene; vinyltoluene; esters of acrylic or methacrylic acid with 1,1-dihydroperfluoroalkanol or with its homologues; esters of acrylic or methacrylic acid with $\omega$-hydrofluoroalkanol; esters of acrylic or methacrylic acid with fluoroalkylsulphoamidoalcohol; esters of acrylic or methacrylic acid with fluoroalkyl alcohol; esters of acrylic or methacrylic acid with alcohol fluoroether; or their mixtures.

4. Composition according to Claim 3, characterized in that the lipophilic monomers (A) are chosen from the group composed of n-butyl methacrylate, isobutyl methacrylate, tert-butyl acrylate, tert-butyl methacrylate, 2-ethylhexyl methacrylate, methyl methacrylate, 2-(N-butylperfluorooctanesulphonamido)ethyl acrylate, 2-(N-methylperfluorooctane-sulphonamido)ethyl acrylate and their mixtures.

5. Composition according to any one of Claims 2 to 4, characterized in that the polar monomers (B) are chosen from the group composed of: acrylic acid, methacrylic acid, N,N-dimethylacrylamide, dimethylaminoethyl methacrylate, quaternized dimethylaminoethyl methacrylate, (meth)acrylamide, N-t-butylacrylamide, maleic acid, maleic anhydride and its half-esters, hydroxyalkyl (meth)acrylates, diallyldimethylammonium chloride, vinylpyrrolidone, vinyl ethers, maleimides, vinylpyridine, vinylimidazole, polar heterocyclic vinyl compounds, styrenesulphonate, allyl alcohol, vinyl alcohol, vinylcaprolactam or their mixtures.

6. Composition according to Claim 5, characterized in that the polar monomers (B) are chosen from the group composed of acrylic acid, N,N-dimethylacrylamide, dimethylaminoethyl methacrylate, quaternized dimethylaminoethyl

methacrylate, vinylpyrrolidone and their mixtures.

**7.** Composition according to any one of Claims 2 to 6, characterized in that the polysiloxane macromer (C) corresponds to the following general formula (II):

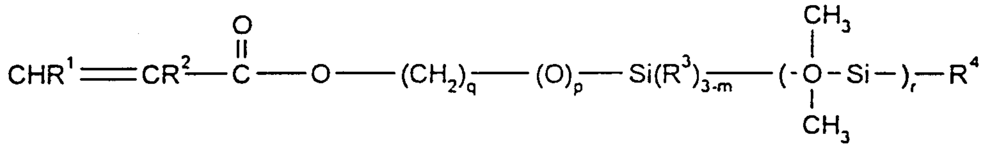

(II)

in which:

$R^1$ is hydrogen or -COOH;
$R^2$ is hydrogen, methyl or $-CH_2COOH$;
$R^3$ is $C_1$-$C_6$ alkyl, alkoxy or alkylamino, $C_6$-$C_{12}$ aryl or hydroxyl;
$R^4$ is $C_1$-$C_6$ alkyl, alkoxy or alkylamino, $C_6$-$C_{12}$ aryl or hydroxyl;
q is an integer from 2 to 6;
p is 0 or 1;
r is an integer from 5 to 700;
m is an integer ranging from 1 to 3.

**8.** Composition according to any one of Claims 2 to 7, characterized in that the polysiloxane macromer (C) corresponds to the following general formula:

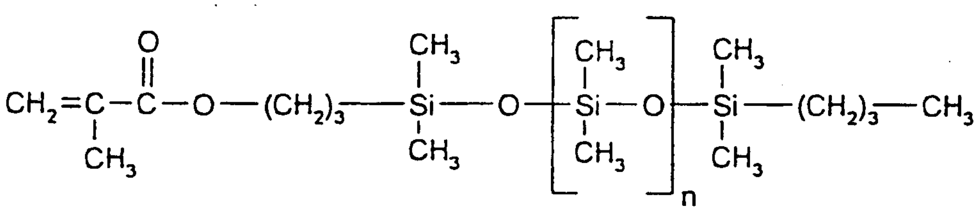

with n being a number ranging from 5 to 700.

**9.** Composition according to any one of Claims 1 to 8, characterized in that the grafted silicone polymer with a non-silicone organic skeleton is a copolymer capable of being obtained by radical polymerization from the mixture of monomers consisting of:

a) 60 weight % of tert-butyl acrylate;
b) 20 weight % of acrylic acid;
c) 20 weight % of silicone macromer of formula:

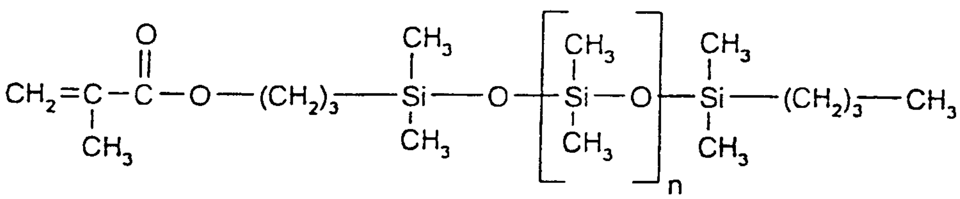

with n being a number ranging from 5 to 700, the percentages by weight being calculated with respect to the total weight of the monomers.

**10.** Composition according to any one of Claims 1 to 8, characterized in that the grafted silicone polymer with a non-silicone organic skeleton is a copolymer capable of being obtained by radical polymerization from the mixture of monomers consisting of:

a) 80 weight % of tert-butyl acrylate;
b) 20 weight % of silicone macromer of formula:

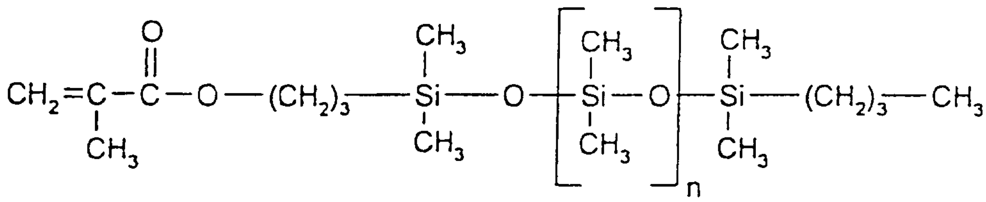

with n being a number ranging from 5 to 700, the percentages by weight being calculated with respect to the total weight of the monomers.

**11.** Composition according to any one of Claims 1 to 10, characterized in that the polymer with a non-silicone organic skeleton grafted by monomers containing a polysiloxane has a number-average molecular weight ranging from 10,000 to 2,000,000 and a glass transition temperature Tg or a crystalline melting temperature Tm of at least -20°C.

**12.** Composition according to Claim 1, characterized in that the grafted silicone polymer with a non-silicone organic skeleton is a polymer capable of being obtained by reactive extrusion of a polysiloxane macromer having an end reactive functional group with a polymer of the polyolefin type containing reactive groups capable of reacting with the end reactive functional group of the polysiloxane macromer in order to form a covalent bond which enables the silicone to be grafted to the main chain of the polyolefin.

**13.** Composition according to Claim 12, characterized in that the reactive polyolefin is chosen from the group composed of polyethylenes or polymers of monomers derived from ethylene containing reactive functional groups capable of reacting with the end functional group of the polysiloxane macromer.

**14.** Composition according to Claim 12 or 13, characterized in that the reactive polyolefin is chosen from the group composed of copolymers of ethylene or of ethylene derivatives and of monomers chosen from those containing a carboxyl functional group; those containing an acid anhydride functional group; those containing an acid chloride functional group; those containing an ester functional group; or those containing an isocyanate functional group.

**15.** Composition according to any one of Claims 12 to 14, characterized in that the polysiloxane macromer is a polysiloxane containing a functionalized group, at the end of the polysiloxane chain or close to the end of the said chain, chosen from the group composed of alcohols, thiols, epoxy groups or primary and secondary amines.

**16.** Composition according to any one of Claims 12 to 15, characterized in that the polysiloxane macromer is a polysiloxane corresponding to the general formula:

$$T\text{-}(CH_2)_s\text{-}Si\text{-}[\text{-}(OSiR^5R^6)_t\text{-}R^7]_y \qquad (III)$$

in which T is chosen from the group composed of $NH_2$, NHR', an epoxy functional group, OH or SH; $R^5$, $R^6$, $R^7$ and R' independently denote a $C_1$-$C_6$ alkyl, phenyl, benzyl or $C_6$-$C_{12}$ alkylphenyl or hydrogen; s is a number from 2 to 100; t is a number from 0 to 1000 and y is a number from 1 to 3.

**17.** Composition according to Claim 1, characterized in that the polymer with a polysiloxane skeleton grafted by a non-silicone organic group is capable of being obtained by radical copolymerization between, on the one hand, (i) at least one anionic non-silicone organic monomer with ethylenic unsaturation chosen, alone or in the form of a mixture of monomers, from linear or branched unsaturated carboxylic acids and/or one hydrophobic non-silicone monomer with ethylenic unsaturation and (ii), on the other hand, a polysiloxane exhibiting, in its chain, at least one or a number of functional groups capable of reacting with the said ethylenic unsaturations of the said non-silicone monomers.

18. Composition according to Claim 17, characterized in that the anionic organic monomer with ethylenic unsaturation is chosen, alone or in the form of a mixture of monomers, from acrylic acid, methacrylic acid, maleic acid, maleic anhydride, itaconic acid, fumaric acid and crotonic acid or their alkali metal, alkaline-earth metal or ammonium salts, or their mixtures.

19. Composition according to Claim 17, characterized in that the hydrophobic organic monomer with ethylenic unsaturation is chosen, alone or as mixtures of monomers, from alkanol acrylic acid esters and/or alkanol methacrylic acid esters, the alkanol preferably being a $C_1$-$C_{18}$ alkanol.

20. Composition according to Claim 19, characterized in that the hydrophobic organic monomer with ethylenic unsaturation is chosen, alone or as a mixture of monomers, from the group composed of isooctyl (meth)acrylate, isononyl (meth)acrylate, 2-ethylhexyl( meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl(meth)acrylate or stearyl (meth)acrylate.

21. Composition according to any one of Claims 1, 17 and 18, characterized in that the grafted polymer with a polysiloxane skeleton comprises, on the main silicone chain, at least one organic group of anionic nature obtained by the radical (homo)polymerization of at least one anionic monomer of unsaturated carboxylic acid type, partially or completely neutralized in the form of a salt.

22. Composition according to any one of Claims 17 to 21, characterized in that the grafted silicone polymer is chosen from the silicone polymers containing, in their structure, the following unit of formula (IV):

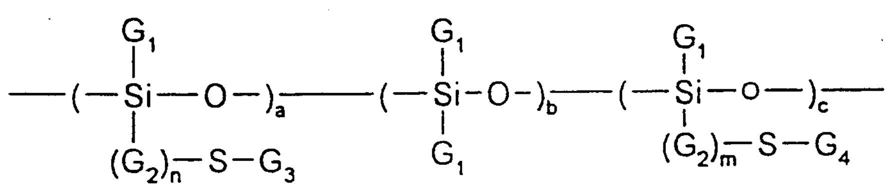

(IV)

in which the $G_1$ radicals, which are identical or different, represent hydrogen or a $C_1$-$C_{10}$ alkyl radical or alternatively a phenyl radical; the $G_2$ radicals, which are identical or different, represent a $C_1$-$C_{10}$ alkylene group; $G_3$ represents a polymer residue resulting from the (homo)polymerization of at least one anionic monomer with ethylenic unsaturation; $G_4$ represents a polymer residue resulting from the (homo)polymerization of at least one hydrophobic monomer with ethylenic unsaturation; m and n are equal to 0 or 1; a is an integer ranging from 0 to 50; b is an integer which can be between 10 and 350 and c is an integer ranging from 0 to 50, with the proviso that one of the parameters a and c is other than 0.

23. Composition according to Claim 22, characterized in that the unit of formula (IV) exhibits at least one of the following characteristics:

- the $G_1$ radicals denote a $C_1$-$C_{10}$ alkyl radical;
- n is not zero and the $G_2$ radicals represent a divalent $C_1$-$C_3$ radical;
- $G_3$ represents a polymer radical resulting from the (homo)polymerization of at least one monomer of the carboxylic acid with ethylenic unsaturation type;
- $G_4$ represents a polymer radical resulting from the (homo)polymerization of at least one monomer of the ($C_1$-$C_{10}$)alkyl (meth)acrylate type.

24. Composition according to Claim 22 or 23, characterized in that the unit of formula (IV) simultaneously exhibits the following characteristics:

- the $G_1$ radicals denote a methyl radical;
- n is not zero and the $G_2$ radicals represent a propylene radical;
- $G_3$ represents a polymer radical resulting from the (homo)polymerization of at least acrylic acid and/or methacrylic acid;
- $G_4$ represents a polymer radical resulting from the (homo)polymerization of at least one monomer of the isobutyl or methyl (meth)acrylate type.

**25.** Composition according to any one of Claims 1 to 24, characterized in that the number-average molecular mass of the polymer with a polysiloxane skeleton grafted by non-silicone organic monomers varies from 10,000 to 1,000,000 approximately and more preferentially still from 10,000 to 100,000 approximately.

**26.** Composition according to any one of Claims 1 to 24, characterized in that the polymer or polymers with a non-silicone organic skeleton grafted by monomers containing a polysiloxane are used in an amount ranging from 0.01 to 20 weight % with respect to the total weight of the composition and preferably from 0.1 to 15 weight % and more preferentially still from 0.5 to 10 %.

**27.** Composition according to any one of Claims 1 to 26, characterized in that the polymer or polymers with a polysiloxane skeleton grafted by non-silicone organic monomers are used in an amount ranging from 0.01 to 20 weight % with respect to the total weight of the composition and preferably from 0.1 to 15 weight % and more preferentially still from 0.5 to 10 %.

**28.** Composition according to any one of Claims 1 to 27, characterized in that it additionally contains at least one additive chosen from the group composed of thickeners, fatty acid esters, esters of fatty acids and of glycerol, silicones, surfactants, fragrances, preservatives, sunscreening agents, proteins, vitamins, polymers, vegetable, animal, mineral or synthetic oils and any other additive conventionally used in the cosmetics field.

**29.** Composition according to any one of Claims 1 to 28, characterized in that the cosmetically or dermatologically acceptable medium is composed of water or a mixture of water and of at least one cosmetically acceptable solvent.

**30.** Composition according to Claim 29, characterized in that the cosmetically acceptable solvents are chosen from the group composed of monoalcohols, polyalcohols, glycol ethers, fatty acid esters and their mixtures.

**31.** Composition according to any one of Claims 1 to 30, characterized in that the polymer with a polysiloxane skeleton grafted by non-silicone organic monomers and the polymer with a non-silicone organic skeleton grafted by monomers containing a polysiloxane can be dissolved in the cosmetically or dermatologically acceptable medium or else used in the form of an agueous dispersion of particles.

**32.** Composition according to any one of Claims 1 to 31, characterized in that the keratinous substances are human hairs.

**33.** Composition according to any one of Claims 1 to 32, characterized in that it is provided in the gel, milk, cream, more or less thickened lotion or foam form.

**34.** Composition according to any one of Claims 1 to 33, characterized in that it is a styling product.

**35.** Composition according to any one of Claims 1 to 33, characterized in that it is a hair product chosen from the group composed of shampoos or hair products which are or are not to be rinsed, to be applied before or after shampooing, dyeing, bleaching, permanent waving or hair straightening.

**36.** Composition according to any one of Claims 1 to 35, characterized in that it is packaged in the form of a vaporizer or pump-action spray or alternatively in an aerosol container for the purpose of obtaining a spray, a lacquer or a foam.

**37.** Non-therapeutic process for the treatment of keratinous substances, in particular hair, characterized in that it comprises the application to the said substances of a composition as defined according to any one of Claims 1 to 36 and then an optional rinsing with water.

**Patentansprüche**

**1.** Kosmetische oder dermatologische Zusammensetzung, die zur Behandlung von Keratinsubstanzen bestimmt ist, dadurch gekennzeichnet, daß sie in einem kosmetisch oder dermatologisch akzeptablen Medium enthält:

a) mindestens ein Polymer mit einem siliconfreien organischen Grundgerüst, auf das ein Polysiloxanmacromer

gepfropft ist, das durch radikalische Polymerisation mindestens eines Monomers mit ethylenisch ungesättigter Doppelbindung mit mindestens einem Polysiloxanmacromer, das eine Vinyl-Endgruppe aufweist, oder auch durch Umsetzung eines Polyolefins, das funktionalisierte Gruppen enthält, mit einem Polysiloxanmacromer mit einer Endgruppe, die mit diesen funktionalisierten Gruppen reagieren kann, erhalten werden kann
und

b) mindestens ein Polymer mit einem Polysiloxangrundgerüst, auf das eine siliconfreie organische Gruppe gepfropft ist, das durch radikalische Copolymerisation von einerseits (i) mindestens einem siliconfreien anionischen organischen Monomer mit ethylenisch ungesättigter Doppelbindung und/oder einem siliconfreien hydrophoben organischen Monomer mit ethylenisch ungesättigter Doppelbindung und andererseits (ii) einem Polysiloxan, das in seiner Kette mindestens eine funktionelle Gruppe oder mehrere funktionelle Gruppen aufweist, die mit den ethylenisch ungesättigten Doppelbindungen der siliconfreien Monomere reagieren können, erhalten werden kann.

**2.** Zusammensetzung nach Anspruch 1, dadurch gekennzeichnet, daß das siliconhaltige Pfropfpolymer mit einem siliconfreien organischen Grundgerüst ein Copolymer ist, umfassend:

a) Null bis 98 Gew.-% mindestens eines schwach polaren lipophilen Monomers (A) mit ethylenisch ungesättigter Doppelbindung, das radikalisch polymerisierbar ist,
b) Null bis 98 Gew.-% mindestens eines polaren hydrophilen Monomers (B) mit ethylenisch ungesättigter Doppelbindung, das mit dem Monomer oder den Monomeren des Typs (A) copolymerisierbar ist,
und
c) 0,01 bis 50 Gew.-% mindestens eines Polysiloxanmacromers (C) der allgemeinen Formel:

$$X(Y)_nSi(R)_{3-m}\text{-}Z_m \qquad (I)$$

worin bedeuten:

- X eine mit den Monomeren (A) und (B) copolymerisierbare Vinylgruppe,
- Y eine zweiwertige Verbindungsgruppe,
- R Wasserstoff, $C_{1-6}$-Alkyl, $C_{1-6}$-Alkyoxy oder $C_{6-12}$-Aryl,
- Z eine einwertige Polysiloxaneinheit mit einem Zahlenmittel des Molekülgewichts von mindestens 500,
- n Null oder 1
  und
- m eine ganze Zahl von 1 bis 3,
  wobei die Prozentanteile auf das Gesamtgewicht der Monomere (A), (B) und (C) bezogen sind.

**3.** Zusammensetzung nach Anspruch 2, dadurch gekennzeichnet, daß die lipophilen Monomere (A) unter den Acrylsäureestern oder Methacrylsäureestern von $C_{1-18}$-Alkoholen, Styrol, den Polystyrolmacromeren, Vinylacetat, Vinylpropionat, $\alpha$-Methylstyrol, *tert.*-Butylstyrol, Butadien, Cyclohexadien, Ethylen, Propylen, Vinyltoluol, den Estern von Acrylsäure oder Methacrylsäure und einem 1,1-Dihydroperfluoralkanol oder seinen Homologen, den Estern von Acrylsäure oder Methacrylsäure und einem $\Omega$-Hydrofluoralkanol, den Estern von Acrylsäure oder Methacrylsäure und einem Fluoralkylsulfonamidoalkohol, den Estern von Acrylsäure oder Methacrylsäure und einem Fluoralkylalkohol, den Estern von Acrylsäure oder Methacrylsäure und einem Alkoholfluorether oder den Gemischen dieser Verbindungen ausgewählt sind.

**4.** Zusammensetzung nach Anspruch 3, dadurch gekennzeichnet, daß die lipophilen Monomere (A) unter n-Butylmethacrylat, Isobutylmethacrylat, *tert.*-Butylacrylat, *tert.* -Butylmethacrylat, 2-Ethylhexylmethacrylat, Methylmethacrylat, 2-(N-Butylperfluoroctansulfonamido)-ethylacrylat, 2-(N-Methylperfluoroctansulfonamido)-ethylacrylat und den Gemischen dieser Verbindungen ausgewählt sind.

**5.** Zusammensetzung nach einem der Ansprüche 2 bis 4, dadurch gekennzeichnet, daß die polaren Monomere (B) ausgewählt sind unter: Acrylsäure, Methacrylsäure, N,N-Dimethylacrylamid, Dimethylaminoethylmethacrylat, quaternisiertem Dimethylaminoethylmethacrylat, (Meth)acrylamid, N-*tert.*-Butylacrylamid, Maleinsäure, Maleinsäureanhydrid und den Halbestern dieser Verbindungen, den Hydroxyalkyl(meth)acrylaten, Diallyldimethylammoniumchlorid, Vinylpyrrolidon, den Vinylethern, den Maleinimiden, Vinylpyridin, Vinylimidazol, den heterocyclischen polaren Vinylverbindungen, Styrolsulfonat, Allylalkohol, Vinylalkohol, Vinylcaprolactam oder den Gemischen dieser Verbindungen.

6. Zusammensetzung nach Anspruch 5, dadurch gekennzeichnet, daß die polaren Monomere (B) unter Acrylsäure, N,N-Dimethylacrylamid, Dimethylaminoethylmethacrylat, quaternisiertem Dimethylaminoethylmethacrylat, Vinylpyrrolidon und den Gemischen dieser Verbindungen ausgewählt sind.

7. Zusammensetzung nach einem der Ansprüche 2 bis 6, dadurch gekennzeichnet, daß das Polysiloxanmacromer (C) der folgenden allgemeinen Formel (II) entspricht:

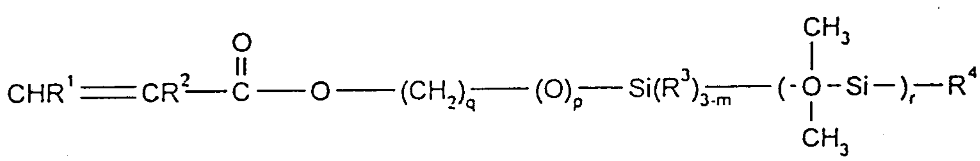

(II)

worin bedeuten:

- $R^1$ Wasserstoff oder -COOH,
- $R^2$ Wasserstoff, Methyl oder $-CH_2COOH$,
- $R^3$ $C_{1-6}$-Alkyl, $C_{1-6}$-Alkoxy, $C_{1-6}$-Alkylamino, $C_{6-12}$-Aryl oder Hydroxy,
- $R^4$ $C_{1-6}$-Alkyl, $C_{1-6}$-Alkoxy, $C_{1-6}$-Alkylamino, $C_{6-12}$-Aryl oder Hydroxy,
- q eine ganze Zahl von 2 bis 6,
- p Null oder 1,
- r eine ganze Zahl von 5 bis 700
  und
- m eine ganze Zahl von 1 bis 3.

8. Zusammensetzung nach einem der Ansprüche 2 bis 7, dadurch gekennzeichnet, daß das Polysiloxanmacromer (C) der folgenden allgemeinen Formel:

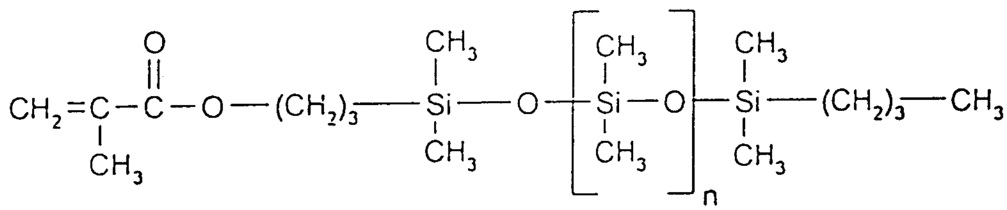

entspricht, wobei n eine Zahl von 5 bis 700 bedeutet.

9. Zusammensetzung nach einem der Ansprüche 1 bis 8, dadurch gekennzeichnet, daß das siliconhaltige Pfropfpolymer mit einem siliconfreien organischen Grundgerüst ein Copolymer ist, das durch radikalische Polymerisation aus dem Monomerengemisch erhalten werden kann, bestehend aus:

a) 60 Gew.-% *tert.*-Butylacrylat,
b) 20 Gew.-% Acrylsäure
und
c) 20 Gew.-% eines siliconhaltigen Macromers der Formel:

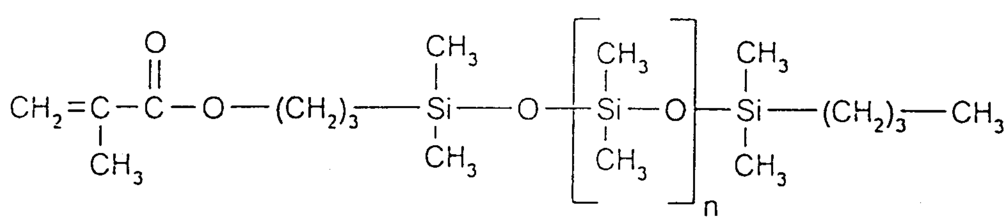

in der n eine Zahl von 5 bis 700 bedeutet, wobei die Prozentanteile auf das Gesamtgewicht der Monomere

bezogen sind.

**10.** Zusammensetzung nach einem der Ansprüche 1 bis 8, dadurch gekennzeichnet, daß das siliconhaltige Pfropfpolymer mit einem siliconfreien organischen Grundgerüst ein Copolymer ist, das durch radikalische Polymerisation aus dem Monomerengemisch erhalten werden kann, bestehend aus:

a) 80 Gew.-% *tert.*-Butylacrylat
und
b) 20 Gew.-% eines siliconhaltigen Macromers der Formel:

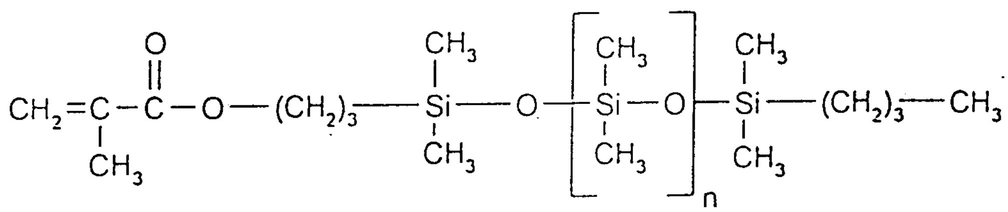

in der n eine Zahl von 5 bis 700 bedeutet, wobei die Prozentanteile auf das Gesamtgewicht der Monomere bezogen sind.

**11.** Zusammensetzung nach einem der Ansprüche 1 bis 10, dadurch gekennzeichnet, daß das Polymer mit einem siliconfreien organischen Grundgerüst, auf das Monomere gepfropft sind, die ein Polysiloxan enthalten, ein Zahlenmittel des Molekülgewichts von 10.000 bis 2.000.000 und eine Glasübergangstemperatur Tg oder eine Schmelztemperatur der Kristallite Tm von mindestens -20°C aufweist.

**12.** Zusammensetzung nach Anspruch 1, dadurch gekennzeichnet, daß das siliconhaltige Pfropfpolymer mit einem siliconfreien organischen Grundgerüst ein Polymer ist, das durch reaktive Extrusion eines Polysiloxanmacromers, das eine reaktive Endgruppe aufweist, mit einem Polymer vom Polyolefintyp erhalten werden kann, welches reaktive Gruppen enthält, die mit der reaktiven Endgruppe des Polysiloxanmacromers unter Bildung einer kovalenten Bindung reagieren können, wodurch das Silicon auf die Hauptkette des Polyolefins gepfropft werden kann.

**13.** Zusammensetzung nach Anspruch 12, dadurch gekennzeichnet, daß das reaktive Polyolefin unter den Polyethylenen oder den Polymeren von Monomeren, die von Ethylen abgeleitet sind, ausgewählt ist, die reaktive Gruppen enthalten, die mit der Endgruppe des Polysiloxanmacromers reagieren können.

**14.** Zusammensetzung nach Anspruch 12 oder 13, dadurch gekennzeichnet, daß das reaktive Polyolefin ausgewählt ist unter den Copolymeren von Ethylen oder Ethylenderivaten und Monomeren, die unter den Monomeren mit einer Carboxygruppe, den Monomeren mit einer Säureanhydridgruppe, den Monomeren mit einer Säurechloridgruppe, den Monomeren mit einer Estergruppe und den Monomeren mit einer Isocyanatgruppe ausgewählt sind.

**15.** Zusammensetzung nach einem der Ansprüche 12 bis 14, dadurch gekennzeichnet, daß das Polysiloxanmacromer ein Polysiloxan ist, das am Ende der Polysiloxankette oder in der Nähe des Endes dieser Kette eine funktionalisierte Gruppe umfaßt, die unter den Alkoholen, den Thiolen, den Epoxygruppen und den primären und sekundären Aminogruppen ausgewählt ist.

**16.** Zusammensetzung nach einem der Ansprüche 12 bis 15, dadurch gekennzeichnet, daß das Polysiloxanmacromer ein Polysiloxan der allgemeinen Formel:

$$T\text{-}(CH_2)_s\text{-}Si\text{-}[\text{-}(OSiR^5R^6)_t\text{-}R^7]_y \qquad (III)$$

worin:

- T unter $NH_2$, NHR', Epoxy, OH und SH ausgewählt ist,
- $R^5$, $R^6$, $R^7$ und R' unabhängig voneinander $C_{1-6}$-Alkyl, Phenyl, Benzyl, $C_{6-12}$-Alkylphenyl oder Wasserstoff bedeuten,
- s eine Zahl von 2 bis 100 bedeutet,
- t eine Zahl von 0 bis 1.000 bedeutet

und

- y eine Zahl von 1 bis 3 bedeutet,

ist.

**17.** Zusammensetzung nach Anspruch 1, dadurch gekennzeichnet, daß das Polymer mit einem Polysiloxangrundgerüst, auf das eine siliconfreie organische Gruppe gepfropft ist, durch radikalische Copolymerisation von einerseits (i) mindestens einem siliconfreien anionischen organischen Monomer mit ethylenisch ungesättigter Doppelbindung, das unter den geradkettigen oder verzweigten ungesättigten Carbonsäuren oder deren Gemischen ausgewählt ist und/oder einem siliconfreien hydrophoben Monomer mit ethylenisch ungesättigter Doppelbindung und von andererseits (ii) einem Polysiloxan, das in seiner Kette mindestens eine funktionelle Gruppe oder mehrere funktionelle Gruppen aufweist, die mit den ethylenisch ungesättigten Doppelbindungen der siliconfreien Monomere reagieren können, erhalten werden kann.

**18.** Zusammensetzung nach Anspruch 17, dadurch gekennzeichnet, daß das anionische organische Monomer mit ethylenisch ungesättigter Doppelbindung unter Acrylsäure, Methacrylsäure, Maleinsäure, Maleinsäureanhydrid, Itaconsäure, Fumarsäure und Crotonsäure oder deren Gemischen oder den Alkalisalzen, den Erdalkalimetallsalzen oder den Ammoniumsalzen dieser Säuren oder den Gemischen dieser Verbindungen ausgewählt ist.

**19.** Zusammensetzung nach Anspruch 17, dadurch gekennzeichnet, daß das hydrophobe organische Monomer mit ethylenisch ungesättigter Doppelbindung unter den Alkanolacrylsäureestern und/oder den Alkanolmethacrylsäureestern oder deren Gemischen ausgewählt ist, wobei das Alkanol vorzugsweise ein $C_{1-18}$-Alkanol ist.

**20.** Zusammensetzung nach Anspruch 19, dadurch gekennzeichnet, daß das hydrophobe organische Monomer mit ethylenisch ungesättigter Doppelbindung unter Isooctyl(meth)acrylat, Isononyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Methyl(meth)acrylat, *tert.*-Butyl(meth)acrylat, Tridecyl(meth)acrylat und Stearyl(meth)acrylat oder den Gemischen dieser Verbindungen ausgewählt ist.

**21.** Zusammensetzung nach einem der Ansprüche 1, 17 und 18, dadurch gekennzeichnet, daß das Pfropfpolymer mit einem Polysiloxangrundgerüst auf seiner Siliconhauptkette mindestens eine organische Gruppe mit anionischem Charakter umfaßt, die durch die radikalische (Homo)polymerisation mindestens eines anionischen Monomers vom Typ einer ungesättigten Carbonsäure, die teilweise oder vollständig in Form eines Salzes neutralisiert ist, erhalten wird.

**22.** Zusammensetzung nach einem der Ansprüche 17 bis 21, dadurch gekennzeichnet, daß das siliconhaltige Pfropfpolymer unter den siliconhaltigen Polymeren ausgewählt ist, die in ihrer Struktur die Einheit der folgenden Formel (IV) aufweisen:

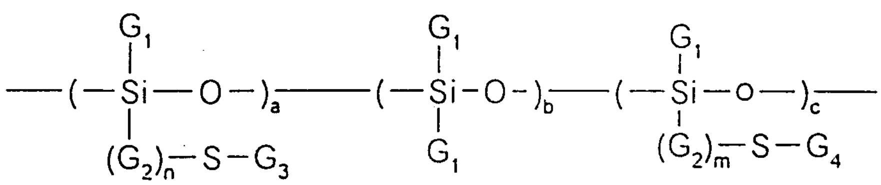

(IV)

worin bedeuten:

- die Gruppen $G_1$, die identisch oder voneinander verschieden sind, Wasserstoff oder eine $C_{1-10}$-Alkylgruppe oder auch eine Phenylgruppe,
- die Gruppen $G_2$, die identisch oder voneinander verschieden sind, eine $C_{1-10}$-Alkylengruppe,
- $G_3$ eine Polymergruppe, die sich aus der (Homo)polymerisation mindestens eines anionischen Monomers mit ethylenisch ungesättigter Doppelbindung ergibt,
- $G_4$ eine Polymergruppe, die sich aus der (Homo)polymerisation mindestens eines hydrophoben Monomers mit ethylenisch ungesättigter Doppelbindung ergibt,
- m und n Null oder 1,
- a Null oder eine ganze Zahl von 1 bis 50,

- b eine ganze Zahl, die im Bereich von 10 bis 350 liegen kann und
- c Null oder eine ganze Zahl von 1 bis 50, mit der Maßgabe, daß einer der Parameter a und c nicht Null ist.

**23.** Zusammensetzung nach Anspruch 22, dadurch gekennzeichnet, daß die Einheit von Formel (IV) mindestens eines der folgenden Merkmale aufweist:

- Die Gruppen G1 bedeuten eine $C_{1-10}$-Alkylgruppe.
- n ist nicht Null, und die Gruppen $G_2$ bedeuten eine zweiwertige Gruppe mit 1 bis 3 Kohlenstoffatomen.
- $G_3$ bedeutet eine Polymergruppe, die sich aus der (Homo)polymerisation mindestens eines Monomers vom Typ einer Carbonsäure mit ethylenisch ungesättigter Doppelbindung ergibt.
- $G_4$ bedeutet eine Polymergruppe, die sich aus der (Homo)polymerisation mindestens eines Monomers vom Typ eines $C_{1-10}$-Alkyl(meth)acrylats ergibt.

**24.** Zusammensetzung nach Anspruch 22 oder 23, dadurch gekennzeichnet, daß die Einheit von Formel (IV) gleichzeitig die folgenden Merkmale aufweist:

- Die Gruppen $G_1$ bedeuten eine Methylgruppe.
- n ist nicht Null, und die Gruppen $G_2$ bedeuten eine Propylengruppe.
- $G_3$ bedeutet eine Polymergruppe, die sich aus der (Homo)polymerisation zumindest von Acrylsäure und/oder Methacrylsäure ergibt.
- $G_4$ bedeutet eine Polymergruppe, die sich aus der (Homo)polymerisation mindestens eines Monomers vom Typ eines Isobutyl(meth)acrylats oder eines Methyl(meth)acrylats ergibt.

**25.** Zusammensetzung nach einem der Ansprüche 1 bis 24, dadurch gekennzeichnet, daß das Zahlenmittel des Molekülgewichts des Polymers mit einem Polysiloxangrundgerüst, auf das siliconfreie organische Monomere gepfropft sind, im Bereich von ungefähr 10.000 bis 1.000.000 und noch bevorzugter im Bereich von ungefähr 10.000 bis 100.000 liegt.

**26.** Zusammensetzung nach einem der Ansprüche 1 bis 24, dadurch gekennzeichnet, daß das Polymer oder die Polymere mit einem siliconfreien organischen Grundgerüst, auf das Monomere gepfropft sind, die ein Polysiloxan enthalten, in einem Mengenanteil von 0,01 bis 20 Gew.-% und vorzugsweise von 0,1 bis 15 Gew.-% und noch bevorzugter von 0,5 bis 10 Gew.-% verwendet werden, bezogen auf das Gesamtgewicht der Zusammensetzung.

**27.** Zusammensetzung nach einem der Ansprüche 1 bis 26, dadurch gekennzeichnet, daß das Polymer oder die Polymere mit einem Polysiloxangrundgerüst, auf das siliconfreie organische Monomere gepfropft sind, in einem Mengenanteil von 0,01 bis 20 Gew.-% und vorzugsweise von 0,1 bis 15 Gew.-% und noch bevorzugter von 0,5 bis 10 Gew.-% verwendet werden, bezogen auf das Gesamtgewicht der Zusammensetzung.

**28.** Zusammensetzung nach einem der Ansprüche 1 bis 27, dadurch gekennzeichnet, daß sie ferner mindestens einen Zusatzstoff enthält, der unter den Verdickungsmitteln, den Fettsäureestern, den Estern von Fettsäuren und Glycerin, den Siliconen, den grenzflächenaktiven Stoffen, den Parfums, den Konservierungsstoffen, den Sonnenschutzfiltern, den Proteinen, den Vitaminen, den Polymeren, den pflanzlichen, tierischen, mineralischen oder synthetischen Ölen oder beliebigen weiteren herkömmlich in der Kosmetik verwendeten Zusatzstoffen ausgewählt ist.

**29.** Zusammensetzung nach einem der Ansprüche 1 bis 28, dadurch gekennzeichnet, daß das kosmetisch oder dermatologisch akzeptable Medium aus Wasser oder einem Gemisch von Wasser und mindestens einem kosmetisch akzeptablen Lösungsmittel besteht.

**30.** Zusammensetzung nach Anspruch 29, dadurch gekennzeichnet, daß die kosmetisch akzeptablen Lösungsmittel unter den Monoalkoholen, den Polyalkoholen, den Glykolethern, den Fettsäureestern und den Gemischen dieser Verbindungen ausgewählt sind.

**31.** Zusammensetzung nach einem der Ansprüche 1 bis 30, dadurch gekennzeichnet, daß das Polymer mit einem Polysiloxangrundgerüst, auf das siliconfreie organische Monomere gepfropft sind, und das Polymer mit einem siliconfreien organischen Grundgerüst, auf das Monomere gepfropft sind, die ein Polysiloxan enthalten, in dem kosmetisch oder dermatologisch akzeptablen Medium gelöst sein können oder auch in Form einer wässerigen

Partikeldispersion verwendet werden können.

**32.** Zusammensetzung nach einem der Ansprüche 1 bis 31, dadurch gekennzeichnet, daß es sich bei den Keratinsubstanzen um menschliches Haar handelt.

**33.** Zusammensetzung nach einem der Ansprüche 1 bis 32, dadurch gekennzeichnet, daß sie in Form eines Gels, einer Milch, einer Creme, einer mehr oder weniger verdickten Lotion oder eines Schaums vorliegt.

**34.** Zusammensetzung nach einem der Ansprüche 1 bis 33, dadurch gekennzeichnet, daß sie ein Frisierprodukt ist.

**35.** Zusammensetzung nach einem der Ansprüche 1 bis 33, dadurch gekennzeichnet, daß es sich um ein Produkt zur Haarbehandlung handelt, das unter Haarwaschmitteln und Haarbehandlungsprodukten zur Anwendung vor oder nach einer Haarwäsche, einer Färbung, einer Entfärbung, einer Dauerwelle oder einer Entkräuselung, die ausgespült werden oder im Haar verbleiben, ausgewählt ist.

**36.** Zusammensetzung nach einem der Ansprüche 1 bis 35, dadurch gekennzeichnet, daß sie in Form eines Zerstäubers, einer Pumpflasche oder auch in einem Aerosolbehälter konfektioniert ist, um ein Spray, einen Lack oder einen Schaum zu erhalten.

**37.** Nichttherapeutisches Verfahren zur Behandlung von Keratinsubstanzen und insbesondere der Haare, dadurch gekennzeichnet, daß es darin besteht, auf diese Substanzen eine nach einem der Ansprüche 1 bis 36 definierte Zusammensetzung aufzutragen und danach gegebenenfalls mit Wasser auszuspülen.